# EUROPEAN PATENT SPECIFICATION

(11) **EP 3 970 799 B1**
(45) Date of publication and mention of the grant of the patent: **11.12.2024**
(21) Application number: 20805337.1
(22) Date of filing: 08.05.2020
(51) Int. Cl.: A61K 31/7034, A61K 31/702, A61K 31/355, A61K 8/60, A61Q 17/00, A61P 17/18, A61P 43/00, A61P 39/06, A61P 35/00, A61K 31/724, A61K 9/00, A61K 9/08, A61K 9/12, A61K 9/70, A61K 31/661, A61K 47/18, A61K 47/32

(54) **INOSITOL DERIVATIVE FOR SUPPRESSING PROLIFERATION OF CANCER CELLS**
INOSITOL-DERIVAT ZUR UNTERDRÜCKUNG DER PROLIFERATION VON KREBSZELLEN
DÉRIVÉ D'INOSITOL POUR INHIBER LA PROLIFERATION DE CELLULES CANCEREUSE

(30) Priority: 13.05.2019 JP 2019090846
(43) Date of publication of application: 23.03.2022
(73) Proprietor: Resonac Corporation, Tokyo (JP)
(72) Inventor: NAKAGAMI Yuko, Tokyo 105-8518 (JP); FUKADA Go, Tokyo 105-8518 (JP); KATO Eiko, Tokyo 105-8518 (JP)
(74) Representative: Strehl Schübel-Hopf & Partner
(86) International application number: PCT/JP2020/018733
(87) International publication number: WO 2020/230741

(56) References cited:
- EP-A1- 3 636 267
- WO-A1-2008/080064
- WO-A1-2018/225718
- WO-A1-2018/225728
- WO-A1-2019/045113
- WO-A2-03/011336
- FR-A1- 2 908 658
- JP-A- 2002 533 475
- JP-A- 2010 514 701
- JP-A- 2011 520 946
- JP-A- S62 294 432
- US-A1- 2019 071 703
- BIZZARRI MARIANO ET AL: "Modulation of both Insulin Resistance and Cancer Growth by Inositol", CURRENT PHARMACEUTICAL DESIGN, vol. 23, no. 34, 18 January 2018 (2018-01-18), NL, XP093039642, ISSN: 1381-6128, DOI: 10.2174/1381612823666170830123634
- BIZZARRI MARIANO ET AL: "Broad Spectrum Anticancer Activity of Myo-Inositol and Inositol Hexakisphosphate", INTERNATIONAL JOURNAL OF ENDOCRINOLOGY, vol. 2016, 1 January 2016 (2016-01-01), US, pages 1 - 14, XP093039640, ISSN: 1687-8337, Retrieved from the Internet <URL:http://downloads.hindawi.com/journals/ije/2016/5616807.pdf> DOI: 10.1155/2016/5616807
- LI, RUYI ET AL.: "Function of PM2.5 in the pathogenesis of lung cancer and chronic airway inflammatory diseases (Review", ONCOLOGY LETTERS, vol. 15, 2018, pages 7506 - 7514, XP55760750, ISSN: 1792-1074
- LIU, QIN ET AL.: "Particulate matter 2.5 regulates lipid synthesis and inflammatory cytokine production in human SZ95 sebocytes", INTERNATIONAL JOURNAL OF MOLECULAR MEDICINE, vol. 40, 2017, pages 1029 - 1036, XP55760753, ISSN: 1107-3756
- YUAN, QI ET AL.: "Ambient fine particulate matter (PM2.5) induces oxidative stress and proinflammatory response via up-regulating the expression of CYP1A1/1B1 in human bronchial epithelial cells in vitro", MU TAT. RES. GEN. TOX. EN., vol. 839, 12 December 2018 (2018-12-12), pages 40 - 48, XP55760756, ISSN: 1383-5718
- LI, JUAN ET AL.: "Amelioration of PM2.5-induced lung toxicity in rats by nutritional supplementation with fish oil and Vitamin E", RESPIRATORY RESEARCH, vol. 20, no. 76, 16 April 2019 (2019-04-16), pages 1 - 9, XP55760758, ISSN: 1465-993X, DOI: https://doi.org/10.1186/s12931-019-1045- 7
- MITKUS, ROBERT J. ET AL.: "Comparative physicochemical and biological characterization of NIST Interim Reference Material PM2.5 and SRM 1648 in human A549 and mouse RAW264.7 cells", TOXICOLOGY IN VITRO, vol. 27, 2013, pages 2289 - 2298, XP55760761, ISSN: 0887-2333

## Description

### [Technical Field]

The present invention relates to a cancer cell proliferation suppression agent and a composition for suppressing proliferation of cancer cells.

Priority is claimed on Japanese Patent Application No. 2019-090846, filed May 13, 2019.

### [Background Art]

In the atmosphere, chemical substances such as aromatic hydrocarbons such as dioxin and PCBs and polycyclic aromatic hydrocarbons (PAHs), oxidizing substances obtained when these chemical substances are oxidized by the action of ultraviolet rays, and the like are present. These substances have various influences on the human body. By inhaling these harmful substances into the human body by exhalation or absorbing them to the mucous membranes, various inflammations occur in the respiratory organs such as the lungs, the mucous membranes of the skin, and the internal organs, and eventually canceration of cells is caused. As a mechanism of carcinogenesis caused by harmful substances such as PAHs contained in this atmospheric dust and oxides thereof, it has been reported that these harmful substances bind to an aryl hydrocarbon receptor (AhR) present in cells, they are transferred into the cell nucleus to induce the expression of a CYP1A1 gene and a CYP1B1 gene, active oxygen (Reactive Oxygen Species: ROS) is produced in cells, and as a result, inflammations, canceration of cells, and invasion of cancer cells are caused (Non Patent Documents 1 and 2).

As agents for defense and agents for protection against atmospheric pollutants and suppression agents of symptoms relating to atmospheric pollution, substances having an antioxidative effect, plant-derived extracts, and the like which are for the purpose of suppressing ROS produced in cells have been reported (Patent Documents 1 to 3). In addition, substances that competitively bind to the AhR to suppress the influences of harmful substances have been reported (Non Patent Document 3). However, this effect is still insufficient.

In addition, as agents for protection for physically repelling atmospheric pollutants, formulations into which an oily coating agent is blended to cover the body surface, agents that capture irritation-causing substances contained in atmospheric pollutants, agents that suppress an oxidizing action, and the like have been proposed. However, although these agents for protection are effective in terms of repelling irritant substances, they are not agents that can protect cells from the in vivo carcinogenic action that may be caused.

### [Citation List]

### [Patent Documents]

[Patent Document 1]
   Japanese Unexamined Patent Application, First Publication No. 2016-88928
[Patent Document 2]
   Japanese Unexamined Patent Application, First Publication No. 2017-186276
[Patent Document 3]
   Japanese Patent No. 6456815

### [Non Patent Documents]

[Non Patent Document 1]
   Moorthy B et al., Polycyclic aromatic hydrocarbons: from metabolism to lung cancer. Toxicol Sci. 2015 May; 145(1):5-15.
[Non Patent Document 2]
   Nebert DW et al., Role of aryl hydrocarbon receptor-mediated induction of the CYP1 enzymes in environmental toxicity and cancer. J Biol Chem. 2004 Jun 4; 279(23):23847-50.
[Non Patent Document 3]
   Furue M et al., Antioxidative Phytochemicals Accelerate Epidermal Terminal Differentiation via the AHR-OVOL1 Pathway: Implications for Atopic Dermatitis. Acta Derm Venereol. 2018 Nov 5; 98(10):918-923.
   Bizzarri Mariano et al., International Journal of Endocrinology, vol. 2016, 1 January 2016, pages 1-14, discloses the broad spectrum anticancer activity of myo-inositol.

### [Summary of Invention]

Note that the references to methods of treatment in the detailed description of the invention in this description are to be interpreted as references to the compounds, pharmaceutical compositions and medicaments of the present invention for use in a method for treatment of the human (or animal) body by therapy.

The present invention relates to an inositol derivative in which glucose or an oligosaccharide containing glucose as a constitutional unit is bound to inositol for use in suppressing cancer cell proliferation in a patient.

### [Technical Problem]

As described above, PAHs in the atmosphere and oxidizing substances thereof are thought to be involved in carcinogenesis, and there is a demand for a drug capable of suppressing the action of these atmospheric pollutants. However, it has not been confirmed that the substances disclosed in Patent Documents 1 to 3 and Non Patent Document 3 have an effect of suppressing the proliferation of cancer cells caused by these atmospheric pollutants.

Accordingly, an object of the present invention is to provide a cancer cell proliferation suppression agent which can suppress proliferation and invasion of cancer cells accelerating due to atmospheric pollutants, and a composition for suppressing proliferation of cancer cells containing the above-mentioned cancer cell proliferation suppression agent.

### [Solution to Problem]

The present invention includes the following aspects.
(1) A cancer cell proliferation suppression agent containing, as an active ingredient, an inositol derivative in which a sugar is bound to inositol, wherein the sugar is glucose or an oligosaccharide containing glucose as a constitutional unit.
(2) The cancer cell proliferation suppression agent according to (1), in which the inositol is myo-inositol.
(3) The cancer cell proliferation suppression agent according to any one of (1) to (2), in which the cancer cell proliferation suppression agent suppresses expression of a CYP1A1 gene and a CYP1B1 gene.
(4) The cancer cell proliferation suppression agent according to any one of (1) to (3), in which the cancer cell proliferation suppression agent suppresses expression of an ARNT gene.
(5) The cancer cell proliferation suppression agent according to any one of (1) to (4), in which the cancer cell proliferation suppression agent suppresses production of active oxygen.
(6) A composition for suppressing proliferation of cancer cells containing: the cancer cell proliferation suppression agent according to any one of (1) to (5); and a pharmaceutically acceptable carrier.
(7) The composition for suppressing proliferation of cancer cells according to (6), in which a total content of the inositol derivative is 0.1 % to 2% by mass.
(8) The composition for suppressing proliferation of cancer cells according to (6) or (7), further containing a tocopherol phosphate ester or a salt thereof.
(9) The composition for suppressing proliferation of cancer cells according to (8), in which the tocopherol phosphate ester is an α-tocopherol phosphate ester.
(10) The composition for suppressing proliferation of cancer cells according to (8) or (9), in which the salt of the tocopherol phosphate ester is a sodium salt of the tocopherol phosphate ester.
(11) The composition for suppressing proliferation of cancer cells according to any one of (8) to (10), in which a total content of the tocopherol phosphate ester or the salt thereof is 0.1% to 2% by mass.

### [Advantageous Effects of Invention]

According to the present invention, it is possible to provide a cancer cell proliferation suppression agent which can suppress proliferation and invasion of cancer cells accelerating due to atmospheric pollutants, and a composition for suppressing proliferation of cancer cells containing the above-mentioned cancer cell proliferation suppression agent.

### [Description of Embodiments]

### [Cancer cell proliferation suppression agent]

In one embodiment, the present invention provides a cancer cell proliferation suppression agent containing, as an active ingredient, an inositol derivative in which glucose or an oligosaccharide containing glucose as a constitutional unit is bound to inositol.

The cancer cell proliferation suppression agent of the present embodiment can be suitably used for suppressing the cell proliferation of cancer cells accelerating due to atmospheric pollutants. In the present specification, the "atmospheric pollutants" mean substances present in the atmosphere and causing a harmful action on the human body, and in particular, substances involved in the development, progression, and the like of cancer. Examples of the harmful action of atmospheric pollutants include carcinogenic actions, cancer cell proliferation promoting actions, and cancer cell invasion promoting actions.

Examples of the atmospheric pollutants include substances included in Standard Reference Material 1648a supplied by the National Institute of Standards and Technology (NIST). Specific examples thereof include polycyclic aromatic hydrocarbons (PAHs), nitro-polycyclic aromatic hydrocarbons (nitro-PAHs), polychlorinated biphenyls (PCBs), and chlorine-based pesticides.

Examples of the polycyclic aromatic hydrocarbons include, but are not limited to, naphthalene, acenaphthene, phenanthrene, methylphenanthrene, anthracene, benzanthracene, dibenzanthracene, fluoranthene, benzofluoranthene, dibenzofluoranthene, pyrene, benzopyrene, dibenzopyrene, perylene, benzoperylene, indenoperylene, chrysene, benzochrysene, triphenylene, picene, coronene, and biphenyl.

Examples of the nitro-polycyclic aromatic hydrocarbons include compounds in which some of hydrogen atoms of the above-exemplified polycyclic aromatic hydrocarbons are substituted with nitro groups. Specific examples thereof include, but are not limited to, 1-nitronaphthalene, 2-nitronaphthalene, 3-nitroacetylene, 4-nitrophenanthrene, 9-nitrophenanthrene, 9-nitroanthracene, 1-nitropyrene, 2-nitropyrene, 4-nitropyrene, 2-nitrofluoranthene, 3-nitrofluoranthene, 8-nitrofluoranthene, 7-nitrobenzanthracene, and 6-nitrochrysene.

Examples of the polychlorinated biphenyls include, but are not limited to, dichlorobiphenyl, trichlorobiphenyl, tetrachlorobiphenyl, pentachlorobiphenyl, hexachlorobiphenyl, heptachlorobiphenyl, octachlorobiphenyl, nonachlorobiphenyl, and decachlorobiphenyl.

Examples of the chlorine-based pesticides include, but are not limited to, benzene hexachloride, hexachlorobenzene, chlordane, Mirex, and dichlorodiphenyltrichloroethane (DDT).

The atmospheric pollutant is not limited to one having a harmful action (carcinogenesis, cancer proliferation, cancer invasion, and the like) alone, and may be one in which a plurality of substances act compositely to exert a harmful action.

As shown in Examples to be described later, when atmospheric pollutants are present, the cell proliferation of cancer cells is promoted. The cancer cell proliferation suppression agent of the present embodiment can effectively suppress the cell proliferation of cancer cells promoted by such atmospheric pollutants. That is, the cancer cell proliferation suppression agent of the present embodiment can suppress the cell proliferation of cancer cells in the presence of atmospheric pollutants as compared to a case in which the cancer cell proliferation suppression agent is not administered.

When a polycyclic aromatic hydrocarbon invades a cell, the polycyclic aromatic hydrocarbon binds to the AhR and is transported into the nucleus by an Aryl Hydrocarbon Receptor Nuclear Translocator (ARNT) to induce the expression of drug metabolizing genes such as CYP1A1 and CYP1B1. ROS is generated intracellularly by the drug metabolism by CYP1A1, CYP1B1, and the like. It has been reported that ROS induces canceration by giving a rise to DNA mutations and/or disturbance of gene expression profiles (Moorthy B et al., Toxicol Sci. 2015 May; 145(1): 5-15.; Nebert DW et al., J Biol Chem. 2004 Jun 4; 279(23): 23847-50.).

As shown in Examples to be described later, the cancer cell proliferation suppression agent of the present embodiment can suppress the expression of a CYP1A1 gene and a CYP1B1 gene induced by atmospheric pollutants. Accordingly, it can also be said that the cancer cell proliferation suppression agent of the present embodiment is an expression suppression agent of the CYP1A1 gene and the CYP1B1 gene. That is, the cancer cell proliferation suppression agent of the present embodiment can suppress the expression of the CYP1A1 gene and the CYP1B1 gene in the presence of atmospheric pollutants as compared to a case in which the cancer cell proliferation suppression agent is not administered.

CYP1A1 (NCBI Gene ID: 1543) is one kind of cytochrome P450 superfamily enzymes which are mono-oxygenases that catalyze reactions involved in drug metabolism. CYP1A1 is localized in the endoplasmic reticulum, is induced to be expressed by a polycyclic aromatic hydrocarbon, and metabolizes the polycyclic aromatic hydrocarbon to generate carcinogenic substances. Examples of base sequences of a human CYP1A1 gene include NM_000499.5, NM_001319216.2, and NM_001319217.2 which are registered in the NCBI Reference Sequence database. The CYP1A1 gene is not limited to those having the above-mentioned sequence, and includes homologs thereof.

CYP1B1 (NCBI Gene ID: 1545) is one kind of cytochrome P450 superfamily enzymes which are mono-oxygenases that catalyze reactions involved in drug metabolism. CYP1B1 is localized in the endoplasmic reticulum, is induced to be expressed by a polycyclic aromatic hydrocarbon, and metabolizes the polycyclic aromatic hydrocarbon to generate carcinogenic substances. Examples of base sequences of a human CYP1B1 gene include NM_000104.3 registered in the NCBI Reference Sequence database. The CYP1B1 gene is not limited to those having the above-mentioned sequence, and includes homologs thereof.

As shown in Examples to be described later, the cancer cell proliferation suppression agent of the present embodiment can suppress the expression of an ARNT gene induced by atmospheric pollutants. Accordingly, it can also be said that the cancer cell proliferation suppression agent of the present embodiment is an expression suppression agent of the ARNT gene. That is, the cancer cell proliferation suppression agent of the present embodiment can suppress the expression of the ARNT gene in the presence of atmospheric pollutants as compared to a case in which the cancer cell proliferation suppression agent is not administered.

ARNT (NCBI Gene ID: 405) is a protein that binds to the AhR, to which a ligand such as a polycyclic aromatic hydrocarbon binds, to transport the ligand-AhR complex to the nucleus. Examples of base sequences a human ARNT gene include NM_001197325.1, 2. NM_001286035.1, 3. NM_001286036.1, 4. NM_001350224.1, 5. NM_001350225.1, 6. NM_001350226.1, 7. NM_001668.4, and 8. NM_178427.2 which are registered in the NCBI Reference Sequence database. The ARNT gene is not limited to those having the above-mentioned sequence, and includes homologs thereof.

As shown in Examples to be described later, the cancer cell proliferation suppression agent of the present embodiment can suppress the production of ROS induced by atmospheric pollutants. Accordingly, it can also be said that the cancer cell proliferation suppression agent of the present embodiment is a production suppression agent of ROS. That is, the cancer cell proliferation suppression agent of the present embodiment can suppress the production of ROS in the presence of atmospheric pollutants as compared to a case in which the cancer cell proliferation suppression agent is not administered.

As shown in Examples to be described later, the cancer cell proliferation suppression agent of the present embodiment can suppress the invasion of cancer cells promoted by atmospheric pollutants. Accordingly, it can also be said that the cancer cell proliferation suppression agent of the present embodiment is an invasion suppression agent of cancer cells. That is, the cancer cell proliferation suppression agent of the present embodiment can suppress the invasion of cancer cells in the presence of atmospheric pollutants as compared to a case in which the cancer cell proliferation suppression agent is not administered.

### (Inositol derivative)

The cancer cell proliferation suppression agent of the present embodiment contains, as an active ingredient, an inositol derivative in which glucose or an oligosaccharide containing glucose as a constitutional unit is bound to inositol.

Inositol is a cyclic hexahydric alcohol represented by C₆H₆(OH)₆. There are nine stereoisomeric forms of inositol: cis-inositol, epi-inositol, allo-inositol, myo-inositol, muco-inositol, neo-inositol, chiro-inositol (D-form and L-form), and scyllo-inositol.

In the cancer cell proliferation suppression agent of the present embodiment, the inositol constitution of the inositol derivative is preferably myo-inositol which is physiologically active among the above-mentioned isomeric forms. Inositol can be synthesized by a method of extraction from rice bran, a chemical synthesis method, a fermentation method, or the like.

In the cancer cell proliferation suppression agent of the present embodiment, the inositol derivative is a compound in which the sugar is bound to a hydroxyl group of inositol. The sugar may be bound to any one of six hydroxyl groups present in an inositol molecule, or may be bound to any two or more thereof.

The sugar bound to inositol may be a monosaccharide or an oligosaccharide. For example, one or more monosaccharides may be bound to one molecule of inositol, one or more oligosaccharides may be bound to one molecule of inositol, or one or more monosaccharides and one or more oligosaccharides may be bound to one molecule of inositol. In the inositol derivative, a total number of monosaccharides or oligosaccharides bound to one molecule of inositol is 1 or more, may be 2 or more for example, may be 3 or more for example, and may be 4 or more for example in terms of monosaccharide units.

In the present specification, a monosaccharide refers to saccharides that cannot be hydrolyzed further, and refers to a compound that is a constituent element when forming a polysaccharide. A monosaccharide can also be said to be the smallest constitutional unit of saccharides. In the present specification, a "monosaccharide unit" refers to a chemical structure corresponding to a monosaccharide. A "monosaccharide unit" can also be said to be a chemical structure derived from a monosaccharide. For example, since a disaccharide is composed of two monosaccharides, it is converted into two monosaccharide units, and since a trisaccharide is composed of three monosaccharides, it is converted into three monosaccharide units. More specifically, for example, since glucose, is composed of one monosaccharide, it is converted into one monosaccharide unit. For example, since α-cyclodextrin is composed of six monosaccharides, it is converted into six monosaccharide units; since β-cyclodextrin is composed of seven monosaccharides, it is converted into seven monosaccharide units; and since γ-cyclodextrin is composed of eight monosaccharides, it is converted into eight monosaccharide units.

The inositol derivative may be a mixture of inositol derivatives in which different numbers of sugars in terms of monosaccharide units are bound to inositol. For example, the inositol derivative may be a mixture of an inositol derivative in which one sugar in terms of monosaccharide units is bound to one molecule of inositol, an inositol derivative in which two sugars in terms of monosaccharide units are bound to one molecule of inositol, an inositol derivative in which three sugars in terms of monosaccharide units are bound to one molecule of inositol, an inositol derivative in which four sugars in terms of monosaccharide units are bound to one molecule of inositol, and an inositol derivative in which five or more sugars in terms of monosaccharide units are bound to one molecule of inositol. For example, the inositol derivative may be one containing 10% to 100% by mass of an inositol derivative, in which two or more sugars in terms of monosaccharide units are bound to one molecule of inositol, with respect to the total mass (100% by mass) of the inositol derivative. The proportion of the inositol derivative, in which two or more sugars in terms of monosaccharide units are bound to one molecule of inositol, to the total mass (100% by mass) of the inositol derivative may be 20% by mass or more, 30% by mass or more, 40% by mass or more, 50% by mass or more, 60% by mass or more, 70% by mass or more, or 80% by mass or more, for example.

The sugar constituting the inositol derivative include glucose, α-cyclodextrin, β-cyclodextrin, and γ-cyclodextrin.

The sugar constituting the inositol derivative is glucose or an oligosaccharide containing glucose as a constitutional unit. The above-mentioned oligosaccharide contains only glucose as a constitutional unit. The molecular weight of the above-mentioned oligosaccharide may be about 300 to 3000, for example. More specific examples of the oligosaccharides include disaccharides such as maltose, trehalose, trisaccharides such as maltotriose:

The inositol derivative may be a mixture of an inositol derivative in which a sugar is a monosaccharide and an inositol derivative in which a sugar is an oligosaccharide. Furthermore, the inositol derivative may be a mixture of inositol derivatives in which different kinds of sugars are bound to inositol.

From the viewpoint of easily obtaining inositol derivatives having a high degree of purification, it is preferable to use β-cyclodextrin which is industrially inexpensive and can be stably supplied as a raw material for inositol derivatives. In this case, the sugar constituting the inositol derivatives contains glucose as a constitutional unit. Meanwhile, when cheaper starch or the like is used as a raw material for inositol derivatives, various sugars are transferred to various places at the time of synthesis of inositol derivatives, and thus a degree of purification of inositol derivatives to be obtained tends to become unstable.

The inositol derivative may be in the form of a pharmaceutically acceptable salt. In the present specification, a "pharmaceutically acceptable salt" means the form of a salt that does not inhibit an effect of suppressing the proliferation of cancer cells which is an effect of the inositol derivative caused by atmospheric pollutants. The pharmaceutically acceptable salts of the inositol derivative are not particularly limited, and examples thereof include salts of alkali metals (sodium, potassium, and the like); salts of alkaline earth metals (magnesium, calcium, and the like); salts of organic bases (pyridine, triethylamine, and the like); salt with amines; salts of organic acids (acetic acid, formic acid, propionic acid, fumaric acid, maleic acid, succinic acid, tartaric acid, citric acid, malic acid, oxalic acid, benzoic acid, methanesulfonic acid, and the like); and salts of inorganic acids (hydrochloric acid, phosphoric acid, hydrobromic acid, sulfuric acid, nitric acid, and the like).

The inositol derivative may be in the form of a solvate. The inositol derivative may be in the form of a solvate of salts of the inositol derivative. The solvate is not particularly limited, but examples thereof include hydrates and ethanol solvates.

### (Method of synthesizing inositol derivative)

A method of synthesizing the inositol derivative is not particularly limited, and the inositol derivative can be appropriately synthesized by a conventionally known method. For example, inositol and cyclodextrin, which is one of oligosaccharides, may be reacted the presence of a cyclodextrin glucanotransferase to synthesize the inositol derivative (refer to, for example, Japanese Unexamined Patent Application, First Publication No. S63-196596). Alternatively, the inositol derivative may be synthesized by a method of obtaining a glucosyl compound using a glucosyl phosphite ester as a sugar donor (refer to, for example, Japanese Unexamined Patent Application, First Publication No. H6-298783).

The cancer cell proliferation suppression agent of the present embodiment may contain one compound, which is selected from the group consisting of the above-mentioned inositol derivatives, salts of the inositol derivatives, and solvates thereof, alone as the inositol derivative, or may contain two or more kinds thereof in combination.

The cancer cell proliferation suppression agent of the present embodiment can be used by administering itself to a patient for the purpose of suppressing cancer cell proliferation induced by atmospheric pollutants. The cancer cell proliferation suppression agent of the present embodiment can also be used by being blended into pharmaceuticals or cosmetic products for the purpose of imparting the function of suppressing cancer cell proliferation induced by atmospheric pollutants. The cancer cell proliferation suppression agent of the present embodiment may be used by being blended into a composition for suppressing proliferation of cancer cells.

The cancer cell proliferation suppression agent of the present embodiment may be used by being administered to a patient before the onset of cancer to prevent cancerous cells from proliferating and cancer from developing. The cancer cell proliferation suppression agent of the present embodiment may be used by being administered to a cancer patient to suppress cancer cell proliferation induced by atmospheric pollutants.

Examples of cancer cells to which the cancer cell proliferation suppression agent of the present embodiment is applied include, but are not limited to, cancer cells of lung cancer, pancreatic cancer, stomach cancer, head and neck cancer, mesothelioma, neuroblastoma, liver cancer, malignant melanoma, uterine cancer, bladder cancer, biliary tract cancer, esophageal cancer, osteosarcoma, testicular tumor, thyroid cancer, acute myeloid leukemia, brain tumor, prostate cancer, squamous cell carcinoma of the head and neck, colon cancer, kidney cancer, ovarian cancer, breast cancer, and the like. The cancer cell proliferation suppression agent of the present embodiment is suitably used for cell proliferation suppression of lung cancer cells.

The cancer cell proliferation suppression agent of the present embodiment can be administered to a patient by the same method as that for a composition for suppressing proliferation of cancer cells to be described later, and is preferably administered transdermally.

### [Composition for suppressing proliferation of cancer cells]

In one embodiment, the present invention provides a composition for suppressing proliferation of cancer cells containing the above-mentioned cancer cell proliferation suppression agent and a pharmaceutically acceptable carrier.

The composition for suppressing proliferation of cancer cells of the present embodiment can be manufactured by mixing the above-mentioned cancer cell proliferation suppression agent, a pharmaceutically acceptable carrier, and optionally other components and formulating them according to a general method (for example, a method described in the Japanese Pharmacopoeia).

In the present specification, a "pharmaceutically acceptable carrier" means a carrier that does not inhibit a physiological activity of an active ingredient and does not exhibit substantial toxicity with respect to its administration target. The phrase "does not exhibit substantial toxicity" means that this component does not exhibit toxicity with respect to an administration target in a generally used dosage. The pharmaceutically acceptable carrier is not particularly limited, and examples thereof includes excipients, binders, disintegrants, lubricants, emulsifiers, stabilizers, diluents, solvents for injections, oily bases, moisturizers, touch sensation improvers, surfactants, polymers, thickeners, gelling agents, solvents, propellants, antioxidants, reducing agents, oxidizing agents, chelating agents, acids, alkali, powders, inorganic salts, water, metal-containing compounds, unsaturated monomers, polyhydric alcohols, polymer additives, adjuvants, wetting agents, tackifiers, oily raw materials, liquid matrices, fat-soluble substances, and polymer carboxylate salts. Specific examples of these components include those described in PCT International Publication No. WO2016/076310. Specific examples of the polymers, thickeners, and gelling agents include methacryloyloxyethyl phosphorylcholine, butyl methacrylate, and polymers thereof. These pharmaceutically acceptable carriers may be used alone or in combination of two or more kinds thereof.

The other components are not particularly limited, but examples thereof include preservatives, antibacterial agents, ultraviolet absorbents, whitening agents, vitamins and derivatives thereof, antiphlogistics, anti-inflammatory agents, hair growth drugs, blood circulation promoters, stimulants, hormones, anti-wrinkle agents, anti-aging agents, tightening agents, cooling sensation agents, warming sensation agents, wound healing promoters, irritation relieving agents, analgesics, cell-activating agents, plant extracts, animal extracts, microbial extracts, seed oils, antipruritic agents, keratin exfoliating and dissolving agents, antiperspirants, refreshing agents, astringents, enzymes, nucleic acids, fragrances, colorants, coloring agents, dyes, pigments, anti-inflammatory analgesics, antifungals, antihistamines, hypnotic sedatives, tranquilizers, antihypertensives, antihypertensive diuretics, antibiotics, anesthetics, antibacterial substances, antiepileptic agents, coronary vasodilators, herbal medicines, anti-itch drugs, keratin softening and exfoliating agents, ultraviolet blockers, antiseptic disinfectants, antioxidant substances, pH adjusters, additives, and metal soaps. Specific examples of these components include those described in PCT International Publication No. WO2016/076310. Specific examples of the plant extracts extracts include Lapsana communis flowers/leaves/stems, and Camellia sinensis leaves. Specific examples of the seed oils include a Moringa oleifera seed oil. Specific examples of the fragrances include perillaldehyde. The other components may be used alone or in combination of two or more kinds thereof.

The composition for suppressing proliferation of cancer cells of the present embodiment can contain the above-mentioned cancer cell proliferation suppression agent in a therapeutically effective amount. The "therapeutically effective amount" means the amount of a drug effective for treating or preventing diseases of patients. The therapeutically effective amount may vary depending on a disease state, age, sex, body weight, and the like of an administration target. In the composition for suppressing proliferation of cancer cells of the present embodiment, the therapeutically effective amount of the above-mentioned cancer cell proliferation suppression agent may be an amount in which an inositol derivative in the cancer cell proliferation suppression agent can suppress cancer cell proliferation promoted by atmospheric pollutants. For example, the therapeutically effective amount of the above-mentioned cancer cell proliferation suppression agent in the composition for suppressing proliferation of cancer cells of the present embodiment may be 0.01% to 20% by mass for example, may be 0.1 % to 10% by mass for example, may be 0.1% to 5% by mass for example, may be 0.1% to 3% by mass for example, may be 0.1% to 2% by mass for example, may be 0.3% to 2% by mass for example, or may be 0.6% to 1.5% by mass for example as the total content of the inositol derivative in the composition.

The content of the inositol derivative in the above-mentioned composition for suppressing proliferation of cancer cells means the content of one kind of inositol derivative when this compound is contained alone, and means the total content of two or more kinds of inositol derivatives when these compounds are contained in combination.

### (Tocopherol phosphate ester)

The composition for suppressing proliferation of cancer cells of the present embodiment may contain tocopherol phosphate ester or a salt thereof as another component.

The inositol derivative suppresses the expression of the CYP1A1 gene, the CYP1B1 gene, and the ARNT gene via AhR. The tocopherol phosphate ester suppresses the production of ROS induced by atmospheric pollutants by an antioxidative effect thereof. Each of the inositol derivative and the tocopherol phosphate ester inhibits different parts of the canceration process. Therefore, it is presumed that the combination use of these will synergistically improve the canceration suppressing effect.

As shown in Examples to be described later, by using the inositol derivative in combination with the tocopherol phosphate ester of a salt thereof, cancer cell proliferation induced by atmospheric pollutants is synergistically suppressed. Therefore, in a preferred aspect, the composition for suppressing proliferation of cancer cells of the present embodiment contains the inositol derivative and the tocopherol phosphate ester or a salt thereof.

As shown in Examples to be described later, by using the inositol derivative in combination with the tocopherol phosphate ester or a salt thereof, ROS production induced by atmospheric pollutants is synergistically suppressed. Therefore, the composition for suppressing proliferation of cancer cells of the present embodiment can also be a composition for suppressing production of ROS containing the inositol derivative and the tocopherol phosphate ester or a salt thereof.

Examples of the tocopherol phosphate ester include a compound represented by General Formula (1). 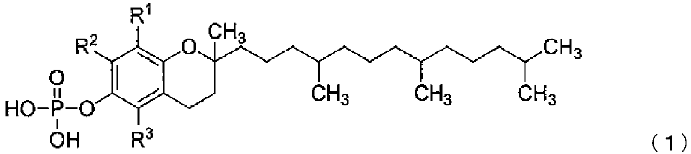 [In the formula, R¹, R², and R³ each independently represent a hydrogen atom or a methyl group.]

As the tocopherol phosphate ester, α-tocopherol phosphate ester (R¹, R², R³ = CH₃), β-tocopherol phosphate ester (R¹, R³ = CH₃, R² = H), γ-tocopherol phosphate ester (R¹, R² = CH₃, R³ = H), δ-tocopherol phosphate ester (R¹ = CH₃, R², R³ = H), ζ₂-tocopherol phosphate ester (R², R³ = CH₃, R¹ = H), η-tocopherol phosphate ester (R² = CH₃, R¹, R³ = H), and the like are present according to the type of R¹, R², and R³ in General Formula (1).

The tocopherol phosphate ester is not particularly limited, and may be any of these tocopherol phosphate esters. Among these, α-tocopherol phosphate ester and γ-tocopherol phosphate ester are preferable, and α-tocopherol phosphate ester is more preferable.

Since the compound represented by General Formula (1) has an asymmetrical carbon atom at the 2-position of a chroman ring, stereoisomeric forms of the d-form and the 1-form, and the dl-form are present. The tocopherol phosphate ester may be any of these stereoisomeric forms, but the dl-form is preferable.

Among the above, as the tocopherol phosphate ester, d1-α-tocopherol phosphate ester and d1-γ-tocopherol phosphate ester are preferable, and d1-α-tocopherol phosphate ester is more preferable.

The salt of the tocopherol phosphate ester is not particularly limited, and examples thereof include salts of inorganic bases and salts of organic bases.

Examples of the salts of inorganic bases include alkali metal salts such as sodium salts and potassium salts; alkaline earth metal salts such as calcium salts and magnesium salts; aluminum salts; ammonium salts; and zinc salts.

Examples of the salts of organic bases include alkylammonium salts and salts of basic amino acids.

Among the above, as the salt of the tocopherol phosphate ester, alkali metal salts are preferable, and sodium salts are more preferable. Among alkali metal salts of the tocopherol phosphate ester, particularly sodium salts have advantages that they are highly soluble in water and they are easily handled because of their property of being a powder.

Examples of preferred forms of the tocopherol phosphate ester include alkali metal salts (for example, sodium salts) of the compound represented by General Formula (1), alkali metal salts (for example, sodium salts) of α-tocopherol phosphate ester, alkali metal salts (for example, sodium salts) of γ-tocopherol phosphate ester, alkali metal salts (for example, sodium salts) of d1-α-tocopherol phosphate ester, and alkali metal salts (for example, sodium salt) of d1-γ-tocopherol phosphate ester.

Sodium salts of d1-α-tocopherol phosphate ester are commercially available from Showa Denko K.K. under the product name of TPNa (registered trademark) (display name: Na tocopheryl phosphate). The above-mentioned TPNa is exemplified as a preferable example of a tocopherol phosphate ester.

In the composition for suppressing proliferation of cancer cells of the present embodiment, one kind selected from a tocopherol phosphate ester and a salt thereof may be used alone, or two or more thereof may be used in combination. The composition for suppressing proliferation of cancer cells of the present embodiment preferably contains salts of tocopherol phosphate ester, and it is more preferable to use alkali metal salts (for example, sodium salts) of tocopherol phosphate ester alone.

The tocopherol phosphate ester or a salt thereof can be manufactured by a known manufacturing method, for example, methods disclosed in Japanese Unexamined Patent Application, First Publication No. S59-44375, WO97/14705, and the like. For example, the tocopherol phosphate ester can be obtained by causing a phosphorylating agent such as phosphorus oxychloride to act on tocopherol dissolved in a solvent and appropriately purifying after completion of the reaction. Furthermore, salts of the obtained tocopherol phosphate ester can be obtained by neutralizing the tocopherol phosphate ester with a metal oxide such as magnesium oxide, a metal hydroxide such as sodium hydroxide, ammonium hydroxide or alkylammonium hydroxide, or the like.

When the composition for suppressing proliferation of cancer cells of the present embodiment contains a tocopherol phosphate ester or a salt thereof, the content of the tocopherol phosphate ester or a salt thereof is not particularly limited. The content of the tocopherol phosphate ester or a salt thereof in the composition for suppressing proliferation of cancer cells of the present embodiment is preferably an amount in which a suppression effect can be synergistically exerted on cancer cell proliferation when used in combination with the inositol derivative. For example, the composition for suppressing proliferation of cancer cells of the present embodiment can contain a therapeutically effective amount of the tocopherol phosphate ester or a salt thereof. The therapeutically effective amount of the tocopherol phosphate ester or a salt thereof in the composition for suppressing proliferation of cancer cells of the present embodiment may be 0.01% to 20% by mass for example, may be 0.1% to 10% by mass for example, may be 0.1% to 5% by mass for example, may be 0.1% to 3% by mass for example, may be 0.1% to 2% by mass for example, may be 0.3% to 2% by mass for example, or may be 0.6% to 1.5% by mass for example as the total content of the tocopherol phosphate ester or a salt thereof in the composition.

The content of the tocopherol phosphate ester or a salt thereof in the above-mentioned composition for suppressing proliferation of cancer cells means the content of one kind of tocopherol phosphate ester of a salt thereof when this compound is contained alone, and means the total content of two or more kinds of tocopherol phosphate esters or salts thereof when these compounds are contained in combination.

The ratio of the inositol derivative to the tocopherol phosphate ester or a salt thereof in the composition for suppressing proliferation of cancer cells of the present embodiment is not particularly limited, but for example, the inositol derivative:the tocopherol phosphate ester of a salt thereof = 1:10 to 10:1 (mass ratio) is possible, 1:5 to 5:1 (mass ratio) is preferable, and 1:3 to 3:1 (mass ratio) is more preferable.

The composition for suppressing proliferation of cancer cells of the present embodiment may be a pharmaceutical composition or a cosmetic preparation.

### (Pharmaceutical composition)

In one embodiment, the present invention provides a pharmaceutical composition for suppressing cancer cell proliferation containing the above-mentioned cancer cell proliferation suppression agent and a pharmaceutically acceptable carrier.

In the pharmaceutical composition of the present embodiment, the pharmaceutically acceptable carrier is not particularly limited, and carriers generally used for pharmaceuticals can be used in addition to the carriers exemplified above. For example, it is possible to use general raw materials described in the Japanese Pharmacopoeia, the Japanese Pharmaceutical Codex, Japanese Pharmaceutical Excipients 2013 (Yakuji Nippo, Ltd., 2013), the Japanese Pharmaceutical Excipients Directory 2016 (edited by International Pharmaceutical Excipients Council Japan, Yakuji Nippo, Ltd., 2016), Handbook of Pharmaceutical Excipients, 7th edition (Pharmaceutical Press, 2012), and the like. These pharmaceutically acceptable carriers may be used alone or in combination of two or more kinds thereof.

The pharmaceutical composition of the present embodiment may contain other components in addition to the above-mentioned cancer cell proliferation suppression agent and the pharmaceutically acceptable carriers. The other components are not particularly limited, and general pharmaceutical additives can be used. Furthermore, as the other components, it is also possible to use an active ingredient other than the above-mentioned cancer cell proliferation suppression agent. As pharmaceutical additives and active ingredients as the other components, in addition to those exemplified above, it is possible to use general raw materials described in, for example, the Japanese Pharmacopoeia, the Japanese Pharmaceutical Codex, Japanese Pharmaceutical Excipients 2013 (Yakuji Nippo, Ltd., 2013), the Japanese Pharmaceutical Excipients Directory 2016 (edited by International Pharmaceutical Excipients Council Japan, Yakuji Nippo, Ltd., 2016), Handbook of Pharmaceutical Excipients, 7th edition (Pharmaceutical Press, 2012), and the like. The other components may be used alone or in combination of two or more kinds thereof. As the other components, tocopherol phosphate esters or salts thereof are preferable exemplary examples.

A dosage form of the pharmaceutical composition of the present embodiment is not particularly limited, and it can be a dosage form generally used for pharmaceutical preparations. Examples thereof include orally administered dosage forms such as tablets, coated tablets, pills, powders, granules, capsules, solutions, suspensions, and emulsions; and parenterally administered dosage forms such as injections, suppositories, external preparations for the skin, and nasal drops. The pharmaceutical composition in these dosage forms can be formulated according to a general method (for example, a method described in the Japanese Pharmacopoeia).

As the pharmaceutical composition of the present embodiment, an external preparation for the skin or a nasal drop is preferable. More specific examples of the external preparation for skin include dosage forms such as creams, lotions, packs, foams, skin cleansers, extracts, plasters, ointments, spirits, suspensions, tinctures, tapes, poultices, liniments, aerosols, sprays, and gels.

A method for administering the pharmaceutical composition of the present embodiment is not particularly limited, and the pharmaceutical composition can be administered by a method generally used as a method for administering pharmaceuticals. For example, it may be administered orally as tablets, coated tablets, pills, powders, granules, capsules, solutions, suspensions, emulsions, and the like; it may be administered intravenously, intraarterially, intramuscularly, intradermally, subcutaneously, intraperitoneally, and the like as an injections, as infusion preparations, and the like alone, or as a mixture with common infusions such as a glucose solution and Ringer's solution; it may be administered rectally as suppositories; it may be administered to skin as external preparations for the skin; or it may be administered intranasally as nasal drops. In a preferred aspect, the pharmaceutical composition of the present embodiment is applied, affixed, or sprayed to an affected area as external preparations for the skin. Alternatively, it is administered intranasally as nasal drops.

The dosage of the pharmaceutical composition of the present embodiment can be a therapeutically effective amount. The therapeutically effective amount may be appropriately determined according to symptoms, body weight, age, sex, and the like of a patient, a dosage form of the pharmaceutical composition, an administration method, and the like. For example, in the case of oral administration, the dosage of the pharmaceutical composition of the present embodiment may be 0.01 to 500 mg per unit of a dosage form as the inositol derivative; in the case of injections, the dosage may be 0.02 to 250 mg per unit of a dosage form as the inositol derivative; in the case of suppositories, the dosage may be 0.01 to 500 mg per unit of a dosage form as the inositol derivative; and the like. In the case of external preparations for the skin or nasal drops, the dosage of the pharmaceutical composition of the present embodiment may be 0.15 to 500 mg per unit of a dosage form as the inositol derivative for example, may be 0.15 to 300 mg for example, may be 0.15 to 200 mg for example, and may be 0.2 to 100 mg for example.

The administration interval of the pharmaceutical composition of the present embodiment may be appropriately determined according to symptoms, body weight, age, sex, and the like of a patient, a dosage form of the pharmaceutical composition, an administration method, and the like. For example, it may be once a day, about 2 to 3 times a day, or the like.

The pharmaceutical composition of the present embodiment can be used by being administered to a cancer patient, for example, to suppress cancer cell proliferation. Furthermore, the pharmaceutical composition of the present embodiment can be used by being administered to a cancer patient to suppress invasion and metastasis of cancer cells.

In urban areas, cancer patients are in daily contact with atmospheric pollutants and are exposed to a risk of promoting cancer cell proliferation. Therefore, it is possible to reduce the risk of promoting cancer cell proliferation due to atmospheric pollutants by administering the pharmaceutical composition of the present embodiment to cancer patients.

Alternatively, in regions in which atmospheric pollutants are present, the pharmaceutical composition of the present embodiment can be used by being prophylactically administered to patients to suppress the proliferation of cancerous cells and prevent the onset of cancer.

### (Cosmetic preparation)

In one embodiment, the present invention provides a cosmetic preparation for suppressing cancer cell proliferation containing the above-mentioned cancer cell proliferation suppression agent and a pharmaceutically acceptable carrier.

In the cosmetic preparation of the present embodiment, the pharmaceutically acceptable carrier is not particularly limited, and carriers generally used for cosmetic preparations can be used in addition to those exemplified above. For example, it is possible to use general raw materials described in the Japanese Standards of Cosmetic Ingredients, Second Edition, Supplements (edited by the Pharmaceutical and Medical Device Regulatory Science Society of Japan, Yakuji Nippo, Ltd., 1984); the Japanese Cosmetic Ingredients Codex (supervisorily edited by the Examination Division of the Pharmaceutical Affairs Bureau, Yakuji Nippo, Ltd., 1993); Supplement to the Japanese Cosmetic Ingredients Codex (supervisorily edited by the Examination Division of the Pharmaceutical Affairs Bureau, Yakuji Nippo, Ltd., 1993); the Comprehensive Licensing Standards of Cosmetics by Category (supervisorily edited by the Examination Division of the Pharmaceutical Affairs Bureau, Yakuji Nippo, Ltd., 1993); the Dictionary of Cosmetic Ingredients (Nikko Chemicals Co., Ltd., 1991); International Cosmetic Ingredient Dictionary and Handbook 2002, Ninth Edition, Vol. 1 to 4, CTFA; and the like. These pharmaceutically acceptable carriers may be used alone or in combination of two or more kinds thereof.

The cosmetic preparation of the present embodiment may contain other components in addition to the cancer cell proliferation suppression agent and the pharmaceutically acceptable carriers. The other components are not particularly limited, and general additives for cosmetic products can be used. Furthermore, as the other components, it is also possible to use an active ingredient other than the above-mentioned cancer cell proliferation suppression agent. As additives for cosmetic products and active ingredients as the other components, in addition to those exemplified above, it is possible to use general raw materials described in, for example, the Japanese Standards of Cosmetic Ingredients, Second Edition, Supplements (edited by the Pharmaceutical and Medical Device Regulatory Science Society of Japan, Yakuji Nippo, Ltd., 1984); the Japanese Cosmetic Ingredients Codex (supervisorily edited by the Examination Division of the Pharmaceutical Affairs Bureau, Yakuji Nippo, Ltd., 1993); Supplement to the Japanese Cosmetic Ingredients Codex (supervisorily edited by the Examination Division of the Pharmaceutical Affairs Bureau, Yakuji Nippo, Ltd., 1993); the Comprehensive Licensing Standards of Cosmetics by Category (supervisorily edited by the Examination Division of the Pharmaceutical Affairs Bureau, Yakuji Nippo, Ltd., 1993); the Dictionary of Cosmetic Ingredients (Nikko Chemicals Co., Ltd., 1991); International Cosmetic Ingredient Dictionary and Handbook 2002, Ninth Edition, Vol. 1 to 4, CTFA; and the like. The other components may be used alone or in combination of two or more kinds thereof. As the other components, tocopherol phosphate esters or salts thereof are preferable exemplary examples.

The form of the cosmetic preparation of the present embodiment is not particularly limited, and it can be a form generally used for cosmetic preparations. Examples thereof include hair cosmetic preparations such as shampoos, hair conditioners, and hairdressing agents; basic cosmetic preparations such as facial cleansers, cleansing agents, skin toners, emulsions, lotions, creams, gels, sunscreens, packs, masks, and serums; makeup cosmetic preparations such as foundations, makeup primers, lipsticks, lip glosses, and blushers; body cosmetic preparations such as body cleansers, body powders, and deodorant cosmetics; and the like. These cosmetic preparations can be manufactured according to a general method. Among these, the cosmetic preparation of the present embodiment is preferably a cosmetic preparation in a form in which it is applied or attached to the skin as an external preparation for the skin. Preferable examples thereof include skin toners, emulsions, lotions, creams, gels, sunscreens, packs, masks, serums, foundations, makeup primers, and the like.

A dosage form of the cosmetic preparation of the present embodiment is not particularly limited, but examples thereof include emulsified types such an oil-in-water (O/W) type, a water-in-oil (W/O) type, a W/O/W type, and an O/W/O type, emulsified polymer types, oily types, solid types, liquid types, kneaded types, stick types, volatile oil types, powder types, jelly types, gel types, paste types, cream types, sheet types, film types, mist types, spray types, multilayer types, foam types, flake types, and the like.

The use amount of the cosmetic preparation of the present embodiment is not particularly limited, but it can be an amount effective for suppressing cancer cell proliferation caused by atmospheric pollutants. For example, the use amount of the cosmetic preparations of the present embodiment may be 0.15 to 500 mg per use as the amount of the inositol derivative, and it may be 0.15 to 300 mg for example, may be 0.15 to 200 mg for example, and may be 0.2 to 100 mg for example.

The use interval of the cosmetic preparation of the present embodiment is not particularly limited, but it can be once a day, about 2 to 3 times a day, or the like, for example.

The cosmetic preparation the present embodiment may be used by cancer patients in urban areas, in which the concentration of atmospheric pollutants is high, and the like to reduce the risk of promoting cancer cell proliferation due to atmospheric pollutants. Alternatively, it may be used in routine skin care and makeup to suppress the proliferation of cancerous cells and prevent the onset of cancer in regions or seasons in which the distribution concentration of atmospheric pollutants is high.

### [Other embodiments]

In one embodiment, the present invention provides a method for suppressing cancer cell proliferation, the method including a step of administering an inositol derivative, in which glucose or an oligosaccharide containing glucose as a constitutional unit is bound to inositol, to a mammal. The above-mentioned cancer cell proliferation is preferably cancer cell proliferation promoted due to atmospheric pollutants.

In one embodiment, the present invention provides a method for suppressing cancer cell invasion, the method including a step of administering an inositol derivative, in which glucose or an oligosaccharide containing glucose as a constitutional unit is bound to inositol, to a mammal. The above-mentioned cancer cell invasion is preferably cancer cell invasion promoted due to atmospheric pollutants.

In one embodiment, the present invention provides a method for suppressing the expression of at least one gene selected from the group consisting of a CYP1 A1 gene, a CYP1B1 gene, and an ARNT gene, the method including a step of administering an inositol derivative, in which glucose or an oligosaccharide containing glucose as a constitutional unit is bound to inositol, to a mammal. The above-mentioned gene expression is preferably gene expression promoted due to atmospheric pollutants.

In one embodiment, the present invention provides a method for suppressing ROS production, the method including a step of administering an inositol derivative, in which glucose or an oligosaccharide containing glucose as a constitutional unit is bound to inositol, to a mammal. The above-mentioned ROS production is preferably ROS production promoted due to atmospheric pollutants.

In one embodiment, the present invention provides an inositol derivative, in which glucose or an oligosaccharide containing glucose as a constitutional unit is bound to inositol, for suppressing cancer cell proliferation. The above-mentioned cancer cell proliferation is preferably cancer cell proliferation promoted due to atmospheric pollutants.

In one embodiment, the present invention provides an inositol derivative, in which glucose or an oligosaccharide containing glucose as a constitutional unit is bound to inositol, for suppressing cancer cell invasion. The above-mentioned cancer cell invasion is preferably cancer cell invasion promoted due to atmospheric pollutants.

In one embodiment, the present invention provides an inositol derivative, in which glucose or an oligosaccharide containing glucose as a constitutional unit is bound to inositol, for suppressing the expression of at least one gene selected from the group consisting of a CYP1A1 gene, a CYP1B1 gene, and an ARNT gene. The above-mentioned gene expression is preferably gene expression promoted due to atmospheric pollutants.

In one embodiment, the present invention provides an inositol derivative, in which glucose or an oligosaccharide containing glucose as a constitutional unit is bound to inositol, for suppressing ROS production. The above-mentioned ROS production is preferably ROS production promoted due to atmospheric pollutants.

In one embodiment, the present invention provides use of an inositol derivative, in which glucose or an oligosaccharide containing glucose as a constitutional unit is bound to inositol, for manufacturing a cancer cell proliferation suppression agent. The above-mentioned cancer cell proliferation suppression agent preferably suppresses cancer cell proliferation promoted due to atmospheric pollutants.

In one embodiment, the present invention provides use of an inositol derivative, in which glucose or an oligosaccharide containing glucose as a constitutional unit is bound to inositol, for manufacturing a cancer cell invasion suppression agent. The above-mentioned cancer cell invasion suppression agent preferably suppresses cancer cell invasion promoted due to atmospheric pollutants.

In one embodiment, the present invention provides use of an inositol derivative, in which glucose or an oligosaccharide containing glucose as a constitutional unit is bound to inositol, for manufacturing an expression suppression agent of at least one gene selected from the group consisting of a CYP1A1 gene, a CYP1B1 gene, and an ARNT gene. The above-mentioned gene expression suppression agent preferably suppresses gene expression promoted due to atmospheric pollutants.

In one embodiment, the present invention provides use of an inositol derivative, in which glucose or an oligosaccharide containing glucose as a constitutional unit is bound to inositol, for manufacturing a ROS production suppression agent. The above-mentioned ROS production suppression agent preferably suppresses ROS production promoted due to atmospheric pollutants.

In one embodiment, the present invention provides use of an inositol derivative, in which glucose or an oligosaccharide containing glucose as a constitutional unit is bound to inositol, for manufacturing a composition for suppressing proliferation of cancer cells. The above-mentioned composition for suppressing proliferation of cancer cells preferably suppresses cancer cell proliferation promoted due to atmospheric pollutants.

In one embodiment, the present invention provides use of an inositol derivative, in which glucose or an oligosaccharide containing glucose as a constitutional unit is bound to inositol, for manufacturing a composition for suppressing invasion of cancer cells. The above-mentioned composition for suppressing invasion of cancer cells preferably suppresses cancer cell invasion promoted due to atmospheric pollutants.

In one embodiment, the present invention provides use of an inositol derivative, in which glucose or an oligosaccharide containing glucose as a constitutional unit is bound to inositol, for manufacturing a composition for suppressing expression of at least one gene selected from the group consisting of a CYP1A1 gene, a CYP1B1 gene, and an ARNT gene. The above-mentioned composition for suppressing expression of a gene preferably suppresses gene expression promoted due to atmospheric pollutants.

In one embodiment, the present invention provides use of an inositol derivative, in which glucose or an oligosaccharide containing glucose as a constitutional unit is bound to inositol, for manufacturing a composition for suppressing production of ROS. The above-mentioned composition for suppressing production of ROS preferably suppresses ROS production promoted due to atmospheric pollutants.

In one embodiment, the present invention provides a method for suppressing cancer cell proliferation, the method including a step of administering an inositol derivative, in which glucose or an oligosaccharide containing glucose as a constitutional unit is bound to inositol, and a tocopherol phosphate ester or a salt thereof to a mammal. The above-mentioned cancer cell proliferation is preferably cancer cell proliferation promoted due to atmospheric pollutants.

In one embodiment, the present invention provides a method for suppressing cancer cell invasion, the method including a step of administering an inositol derivative, in which glucose or an oligosaccharide containing glucose as a constitutional unit is bound to inositol, and a tocopherol phosphate ester of a salt thereof to a mammal. The above-mentioned cancer cell invasion is preferably cancer cell invasion promoted due to atmospheric pollutants.

In one embodiment, the present invention provides a method for suppressing the expression of at least one gene selected from the group consisting of a CYP1A1 gene, a CYP1B1 gene, and an ARNT gene, the method including a step of administering an inositol derivative, in which glucose or an oligosaccharide containing glucose as a constitutional unit is bound to inositol, and a tocopherol phosphate ester or a salt thereof to a mammal. The above-mentioned gene expression is preferably gene expression promoted due to atmospheric pollutants.

In one embodiment, the present invention provides a method for suppressing ROS production, the method including a step of administering an inositol derivative, in which glucose or an oligosaccharide containing glucose as a constitutional unit is bound to inositol, and a tocopherol phosphate ester or a salt thereof to a mammal. The above-mentioned ROS production is preferably ROS production promoted due to atmospheric pollutants.

In one embodiment, the present invention provides a combination of an inositol derivative in which glucose or an oligosaccharide containing glucose as a constitutional unit is bound to inositol, and a tocopherol phosphate ester or a salt thereof, for suppressing cancer cell proliferation. The above-mentioned cancer cell proliferation is preferably cancer cell proliferation promoted due to atmospheric pollutants.

In one embodiment, the present invention provides a combination of an inositol derivative, in which glucose or an oligosaccharide containing glucose as a constitutional unit is bound to inositol, and a tocopherol phosphate ester or a salt thereof, for suppressing cancer cell invasion. The above-mentioned cancer cell invasion is preferably cancer cell invasion promoted due to atmospheric pollutants.

In one embodiment, the present invention provides a combination of an inositol derivative, in which glucose or an oligosaccharide containing glucose as a constitutional unit is bound to inositol, and a tocopherol phosphate ester or a salt thereof, for suppressing the expression of at least one gene selected from the group consisting of a CYP1A1 gene, a CYP1B1 gene, and an ARNT gene. The above-mentioned gene expression is preferably gene expression promoted due to atmospheric pollutants.

In one embodiment, the present invention provides a combination of an inositol derivative, in which glucose or an oligosaccharide containing glucose as a constitutional unit is bound to inositol, and a tocopherol phosphate ester or a salt thereof, for suppressing ROS production. The above-mentioned ROS production is preferably ROS production promoted due to atmospheric pollutants.

In one embodiment, the present invention provides use of a combination of an inositol derivative, in which glucose or an oligosaccharide containing glucose as a constitutional unit is bound to inositol, and a tocopherol phosphate ester or a salt thereof, for manufacturing a cancer cell proliferation suppression agent. The above-mentioned cancer cell proliferation suppression agent preferably suppresses cancer cell proliferation promoted due to atmospheric pollutants.

In one embodiment, the present invention provides use of a combination of an inositol derivative, in which glucose or an oligosaccharide containing glucose as a constitutional unit is bound to inositol, and a tocopherol phosphate ester or a salt thereof, for manufacturing a cancer cell invasion suppression agent. The above-mentioned cancer cell invasion suppression agent preferably suppresses cancer cell invasion promoted due to atmospheric pollutants.

In one embodiment, the present invention provides use of a combination of an inositol derivative, in which glucose or an oligosaccharide containing glucose as a constitutional unit is bound to inositol, and a tocopherol phosphate ester or a salt thereof, for manufacturing an expression suppression agent of at least one gene selected from the group consisting of a CYP1A1 gene, a CYP1B1 gene, and an ARNT gene. The above-mentioned gene expression suppression agent preferably suppresses gene expression promoted due to atmospheric pollutants.

In one embodiment, the present invention provides use of a combination of an inositol derivative, in which glucose or an oligosaccharide containing glucose as a constitutional unit is bound to inositol, and a tocopherol phosphate ester or a salt thereof, for manufacturing a ROS production suppression agent. The above-mentioned ROS production suppression agent preferably suppresses ROS production promoted due to atmospheric pollutants.

In one embodiment, the present invention provides use of a combination of an inositol derivative, in which glucose or an oligosaccharide containing glucose as a constitutional unit is bound to inositol, and a tocopherol phosphate ester or a salt thereof, for manufacturing a composition for suppressing proliferation of cancer cells. The above-mentioned composition for suppressing proliferation of cancer cells preferably suppresses cancer cell proliferation promoted due to atmospheric pollutants.

In one embodiment, the present invention provides use of a combination of an inositol derivative, in which glucose or an oligosaccharide containing glucose as a constitutional unit is bound to inositol, and a tocopherol phosphate ester or a salt thereof, for manufacturing a composition for suppressing invasion of cancer cells. The above-mentioned composition for suppressing invasion of cancer cells preferably suppresses cancer cell invasion promoted due to atmospheric pollutants.

In one embodiment, the present invention provides use of a combination of an inositol derivative, in which glucose or an oligosaccharide containing glucose as a constitutional unit is bound to inositol, and a tocopherol phosphate ester or a salt thereof, for manufacturing a composition for suppressing expression of at least one gene selected from the group consisting of a CYP1A1 gene, a CYP1B1 gene, and an ARNT gene. The above-mentioned composition for suppressing expression of a gene preferably suppresses gene expression promoted due to atmospheric pollutants.

In one embodiment, the present invention provides use of a combination of an inositol derivative, in which glucose or an oligosaccharide containing glucose as a constitutional unit is bound to inositol, and a tocopherol phosphate ester or a salt thereof, for manufacturing a composition for suppressing production of ROS. The above-mentioned composition for suppressing production of ROS preferably suppresses ROS production promoted due to atmospheric pollutants.

### [Examples]

Hereinafter, the present invention will be described with reference to examples, but the present invention is not limited to the following examples.

### [Manufacturing example of inositol derivative]

Myo-inositol and β-cyclodextrin were reacted in the presence of cyclodextrin glucanotransferase to produce an inositol derivative in which glucose or an oligosaccharide having glucose as a monosaccharide unit was bound to myo-inositol. As a result of analyzing the produced inositol derivative by liquid chromatography-mass spectrometry (LC-MS), the proportion of molecules in which the number of glucose of a glucose chain bound to myo-inositol was one was 12% by mass, the proportion of molecules in which the number thereof was two was 30% by mass, the proportion of molecules in which the number thereof was three was 9% by mass, the proportion of molecules in which the number thereof was four was 12% by mass, and the proportion of molecules in which the number thereof was five was 2% by mass.

In the following experimental examples, the inositol derivative manufactured in the present manufacturing example was used.

### [Experimental Example 1]

### (Effect of suppressing expression of CYP1A1 gene and CYP1B1 gene)

The effect of the inositol derivative on suppressing the expression of a CYP1A1 gene and a CYP1B1 gene in normal human epidermal keratinocytes (NHEK, manufactured by KURABO INDUSTRIES LTD.) was measured under the following conditions.

The NHEK cells were seeded in a HuMedia KG2 medium manufactured by KURABO INDUSTRIES LTD. at the seeding density of 10000 cells/cm², and cultured for 24 hours under the conditions of 37°C and 5% CO₂. Next, an aqueous solution of the inositol derivative or an aqueous solution of myo-inositol was added to the culture medium so that the final concentration of the inositol derivative or myo-inositol was 0.001 % by mass, or water (pure water) was added to the culture medium, and culturing was further performed for 24 hours. Thereafter, 0.1 mL of a DMSO solution of atmospheric dust (NIST 1648a) was added per 100 mL of the culture medium so that the final concentration of the atmospheric dust in the culture medium was 500 µg/mL, and culturing was further performed for 48 hours. Thereafter, the NHEK cells were recovered to extract total RNA using Nucleospin^{™} RX (Takara Bio Inc.). From the obtained RNAs, cDNA was synthesized using a PrimeScript (registered trademark) RT Master Mix (Takara Bio Inc.). Using this cDNA as a template, the expression levels of the CYP1A1 gene and the CYP1B1 gene were quantitatively determined by quantitative real-time PCR using primers (QuantiTect Primer Assays, manufactured by QIAGEN) specific to the CYP1A1 gene and the CYP1B1 gene. As an internal standard gene, the expression level of a GAPDH gene, which is a housekeeping gene in which expression fluctuation due to the addition of atmospheric dust is not shown, was quantitatively determined (primer used: Perfect Real Time Primer, manufactured by Takara Bio Inc.), and the expression levels of the CYP1A1 gene and the CYP1B1 gene were standardized based on the expression level of GAPDH. One to which atmospheric dust was not added was used as a control.

Table 1 shows the results. Table 1 shows the expression level of each gene in the atmospheric dust-added group as a relative expression level when the expression level of each gene in the control to which atmospheric dust was not added was 1. In the inositol derivative-added group, the expression levels of both the CYP1A1 gene and the CYP1B1 gene were reduced as compared to the water-added group and the myo-inositol-added group. From these results, it was confirmed that the inositol derivative has a high suppression effect on the expression of the CYP1A1 gene and the CYP1B1 gene induced by atmospheric dust.

**[Table 1]**

| Sample | | Relative gene expression level | |
|---|---|---|---|
| | | CYP1A1 | CYP1B1 |
| Atmospheric dust not added | Water | 1.00 | 1.00 |
| Atmospheric dust added | Water | 4.11 | 4.30 |
| | Inositol derivative | 0.57 | 0.41 |
| | myo-Inositol | 3.10 | 1.08 |

### [Experimental Example 2]

### (Effect of suppressing expression of ARNT gene)

The effect of the inositol derivative on suppressing the expression of an ARNT gene in normal human epidermal keratinocytes (NHEK, manufactured by KURABO INDUSTRIES LTD.) was measured under the following conditions.

The NHEK cells were seeded in a HuMedia KG2 medium manufactured by KURABO INDUSTRIES LTD. at the seeding density of 10000 cells/cm², and cultured for 24 hours under the conditions of 37°C and 5% CO₂. Next, an aqueous solution of the inositol derivative or an aqueous solution of myo-inositol was added to the culture medium so that the final concentration of the inositol derivative or myo-inositol was 0.001% by mass, or water (pure water) was added to the culture medium, and culturing was further performed for 24 hours. Thereafter, 0.1 mL of a DMSO solution of atmospheric dust (NIST 1648a) was added per 100 mL of the culture medium so that the final concentration of the atmospheric dust in the culture medium was 500 µg/mL, and culturing was further performed for 48 hours. Thereafter, the NHEK cells were recovered to extract total RNA using Nucleospin^{™} RX (Takara Bio Inc.). From the obtained RNAs, cDNA was synthesized using a PrimeScript (registered trademark) RT Master Mix (Takara Bio Inc.). Using this cDNA as a template, the expression level of the ARNT gene was quantitatively determined by quantitative real-time PCR using a primer specific to the ARNT gene (Perfect Real Time Primer, manufactured by Takara Bio Inc.). As an internal standard gene, the expression level of GAPDH was quantitatively determined (primer used: Perfect Real Time Primer, manufactured by Takara Bio Inc.), and the expression level of the ARNT gene was standardized based on the expression level of GAPDH. One to which atmospheric dust was not added was used as a control.

Table 2 shows the results. Table 2 shows the expression level of the ARNT gene in the atmospheric dust-added group as a relative expression level when the expression level of the ARNT gene in the control to which atmospheric dust was not added was 1. In the inositol derivative-added group, the expression level of the ARNT gene was reduced as compared to the water-added group and the myo-inositol-added group. From these results, it was confirmed that the inositol derivative has a high suppression effect on the expression of the ARNT gene induced by atmospheric dust.

**[Table 2]**

| Sample | | Relative gene expression level |
|---|---|---|
| | | ARNT |
| Atmospheric dust not added | Water | 1.00 |
| Atmospheric dust added | Water | 1.74 |
| | Inositol derivative | 0.36 |
| | myo-Inositol | 1.85 |

### [Experimental Example 3]

### (Effect (1) of suppressing ROS production)

The effect of the inositol derivative on suppressing ROS production in normal human epidermal keratinocytes (NHEK, manufactured by KURABO INDUSTRIES LTD.) was measured under the following conditions.

The NHEK cells were seeded in a HuMedia KG2 medium manufactured by KURABO INDUSTRIES LTD. at the seeding density of 10000 cells/cm², and cultured for 24 hours under the conditions of 37°C and 5% CO₂. Next, an aqueous solution of the inositol derivative or an aqueous solution of myo-inositol was added to the culture medium so that the final concentration of the inositol derivative or myo-inositol was 0.001% by mass, or water (pure water) was added to the culture medium, and culturing was further performed for 24 hours. Thereafter, 0.1 mL of a DMSO solution of atmospheric dust (NIST 1648a) was added per 100 mL of the culture medium so that the final concentration of the atmospheric dust in the culture medium was 500 µg/mL, and culturing was further performed for 48 hours in the same conditions as above. Thereafter, the ROS production amount was measured using a ROS assay kit (manufactured by OZ BIOSCIENCES). After washing the cells from which the culture medium was removed with a phosphate buffer solution (PBS, manufactured by FUJIFILM Wako Pure Chemical Corporation), 100 µL of dichlorofluorescein diacetate attached to the ROS assay kit was added to each group of the cells, and the cells were left to stand at 37°C for 30 minutes while being shielded from light. After washing the cells with PBS again, 100 µL of PBS was added, and the fluorescence intensity at an excitation wavelength of 485 nm/an absorption wavelength of 535 nm was measured with a microplate reader i-Control (manufactured by Tecan Group Ltd.). One to which atmospheric dust was not added was used as a control.

Table 3 shows the results. Table 3 shows the ROS production amount in the atmospheric dust-added group as a relative amount when the fluorescence intensity in the control to which atmospheric dust was not added was 1. In the inositol derivative-added group, the ROS production amount was reduced as compared to the water-added group and the myo-inositol-added group. From these results, it was confirmed that the inositol derivative has a high suppression effect on the ROS production induced by atmospheric dust. On the other hand, in the myo-inositol-added group, the effect of suppressing ROS production was not recognized as compared to the water-added group.

**[Table 3]**

| Sample | | Relative ROS production amount |
|---|---|---|
| Atmospheric dust not added | Water | 1.00 |
| Atmospheric dust added | Water | 1.46 |
| | Inositol derivative | 1.19 |
| | myo-Inositol | 1.83 |

### [Experimental Example 4]

### (Effect (1) of suppressing proliferation of cancer cells)

The effect of the inositol derivative on suppressing the proliferation of cancer cells in a cell line derived from Lewis lung carcinoma (LLC, JCRB Cell Bank) was measured under the following conditions.

The LLC cells were seeded at the seeding density of 50000 cells/cm² in a culture medium in which a Ham F10 medium and an L15 medium (both manufactured by Sigma-Aldrich) were mixed at the ratio of 3:7 (volume ratio), and cultured for 24 hours under the conditions of 37°C and 5% CO₂. Next, an aqueous solution of the inositol derivative or an aqueous solution of myo-inositol was added to the culture medium so that the final concentration of the inositol derivative or myo-inositol was 0.001% by mass, or water (pure water) was added to the culture medium, and culturing was further performed for 24 hours. Thereafter, 0.1 mL of a DMSO solution of atmospheric dust (NIST 1648a) was added per 100 mL of the culture medium so that the final concentration of the atmospheric dust in the culture medium was 500 µg/mL, and culturing was further performed for 48 hours in the same conditions as above. Thereafter, the culture medium was replaced with a medium containing 10% (V/V) of WST-8 of Nacalai Tesque Inc., and after further culturing for 3 hours, the absorbance at the wavelength of 450 nm was measured with a microplate reader i-Control (manufactured by Tecan Group Ltd.). One to which atmospheric dust was not added was used as a control.

Table 4 shows the results. Table 4 shows the cell proliferation amount in the atmospheric dust-added group as a relative amount when the absorbance in the control to which atmospheric dust was not added was 1. In the inositol derivative-added group, the cell proliferation amount of cancer cells was reduced as compared to the water-added group and the myo-inositol-added group. From these results, it was confirmed that the inositol derivative has a high suppression effect on the cell proliferation of cancer cells accelerating due to atmospheric dust.

**[Table 4]**

| | | Relative cell proliferation amount |
|---|---|---|
| Atmospheric dust not added | Water | 1.00 |
| Atmospheric dust added | Water | 2.31 |
| | Inositol derivative | 1.13 |
| | myo-Inositol | 2.03 |

### [Experimental Example 5]

### (Effect of suppressing invasion of cancer cells)

The effect of the inositol derivative on suppressing the invasion of cancer in a cell line derived from Lewis lung carcinoma (LLC, JCRB Cell Bank) was measured under the following conditions. A CytoSelect invasion assay kit manufactured by Cell Biolabs, Inc. was used for a test.

The LLC cells were seeded in a chamber plate for invasion tests attached to the above-mentioned invasion assay kit so that the concentration was 100000 cells/mL using a culture medium in which a Ham F10 medium and an L15 medium (both manufactured by Sigma-Aldrich) were mixed at the ratio of 3:7 (volume ratio), and cultured for 24 hours under the conditions of 37°C and 5% CO₂. Next, an aqueous solution of the inositol derivative or an aqueous solution of myo-inositol was added to the culture medium so that the final concentration of the inositol derivative or myo-inositol was 0.001% by mass, or water (pure water) was added to the culture medium, and culturing was further performed for 24 hours. Thereafter, 0.1 mL of a DMSO solution of atmospheric dust (NIST 1648a) was added per 100 mL of the culture medium so that the final concentration of the atmospheric dust in the culture medium was 500 µg/mL, and culturing was further performed for 6 hours. Next, the medium containing atmospheric dust was removed and replaced with a new medium, 0.1 mL of a DMSO solution of atmospheric dust (NIST 1648a) was added per 100 mL of the culture medium so that the final concentration of the atmospheric dust in the culture medium was 500 µg/mL, and culturing was further performed for 18 hours. Thereafter, the cells were stained using the above-mentioned invasion assay kit, and the fluorescence intensity at an excitation wavelength of 480 nm/an absorption wavelength of 570 nm was measured with a microplate reader i-Control (manufactured by Tecan Group Ltd.). One to which atmospheric dust was not added was used as a control.

Table 5 shows the results. Table 5 shows the cell invasion in the atmospheric dust-added group as a relative amount when the fluorescence intensity in the control to which atmospheric dust was not added was 1. In the inositol derivative-added group, the cell invasion of cancer cells was reduced as compared to the water-added group and the myo-inositol-added group. From these results, it was confirmed that the inositol derivative suppresses the cell invasion of cancer cells accelerating due to atmospheric dust.

**[Table 5]**

| | | Relative cell invasion |
|---|---|---|
| Atmospheric dust not added | Water | 1.00 |
| Atmospheric dust added | Water | 1.67 |
| | Inositol derivative | 1.23 |
| | myo-Inositol | 1.53 |

### [Experimental Example 6]

### (Effect (2) of suppressing ROS production)

The effect of the inositol derivative and Na tocopherol phosphate on suppressing ROS production in normal human epidermal keratinocytes (NHEK, manufactured by KURABO INDUSTRIES LTD.) was measured under the following conditions. As the following Na tocopherol phosphate, TPNa (registered trademark) (manufactured by Showa Denko K.K.), which is sodium dl-α-tocopheryl phosphate, was used.

The NHEK cells were seeded in a HuMedia KG2 medium manufactured by KURABO INDUSTRIES LTD. at the seeding density of 10000 cells/cm², and cultured for 24 hours under the conditions of 37°C and 5% CO₂. Next, Na tocopherol phosphate alone (final concentration of 10 µM in the culture medium), the inositol derivative alone (final concentration of 0.001% by mass in the culture medium), or a combination of Na tocopherol phosphate (final concentration of 10 µM in the culture medium) and the inositol derivative (final concentration of 0.001% by mass in the culture medium) was added to the culture medium. The Na tocopherol phosphate and the inositol derivative were dissolved in an aqueous solution of 0.05% (V/V) ethanol and added to the culture medium so that the final concentrations were as described above. Furthermore, one in which an aqueous solution of 0.05% (V/V) ethanol was added to the culture medium was also prepared. Thereafter, culturing was performed for 24 hours under the conditions of 37°C and 5% CO₂. Thereafter, 0.1 mL of a DMSO solution of atmospheric dust (NIST 1648a) was added per 100 mL of the culture medium so that the final concentration of the atmospheric dust in the culture medium was 500 µg/mL, and culturing was further performed for 48 hours. Thereafter, the ROS production amount was measured using a ROS assay kit (manufactured by OZ BIOSCIENCES). After washing the cells from which the culture medium was removed with a phosphate buffer solution (PBS, manufactured by FUJIFILM Wako Pure Chemical Corporation), 100 µL of dichlorofluorescein diacetate attached to the ROS assay kit was added to each group of the cells, and the cells were left to stand at 37°C for 30 minutes while being shielded from light. After washing the cells with PBS again, 100 µL of PBS was added, and the fluorescence intensity at an excitation wavelength of 485 nm/an absorption wavelength of 535 nm was measured with a microplate reader i-Control (manufactured by Tecan Group Ltd.). One to which atmospheric dust was not added was used as a control.

Table 6 shows the results. Table 6 shows the ROS production amount in the atmospheric dust-added group as a relative amount when the fluorescence intensity in the control to which atmospheric dust was not added was 1. In any of the group in which the inositol derivative was added alone, the group in which the Na tocopherol phosphate was added alone, and the inositol derivative + Na tocopherol phosphate-added group, the ROS production amount was reduced as compared to the 0.05% ethanol-added group. In the inositol derivative + Na tocopherol phosphate-added group, the production of ROS was reduced than the group in which the inositol derivative was added alone and the group in which the Na tocopherol phosphate was added alone. From these results, it was confirmed that a synergistic suppression effect of the inositol derivative on ROS production induced by atmospheric dust is obtained when the inositol derivative is used together with Na tocopherol phosphate.

**[Table 6]**

| Sample | | Relative ROS production amount |
|---|---|---|
| Atmospheric dust not added | 0.05% Ethanol | 1.00 |
| Atmospheric dust added | 0.05% Ethanol | 2.41 |
| | Na tocopherol phosphate | 1.53 |
| | Inositol derivative | 1.73 |
| | Na tocopherol phosphate + inositol derivative | 1.04 |

### [Experimental Example 7]

### (Effect (2) of suppressing proliferation of cancer cells)

The effect of the inositol derivative and Na tocopherol phosphate on suppressing the proliferation of cancer cells in a cell line derived from Lewis lung carcinoma (LLC, JCRB Cell Bank) was measured under the following conditions. As the following Na tocopherol phosphate, TPNa (registered trademark) (manufactured by Showa Denko K.K.), which is sodium dl-α-tocopheryl phosphate, was used.

The LLC cells were seeded at the seeding density of 50000 cells/cm² in a culture medium in which a Ham F10 medium and an L15 medium (both manufactured by Sigma-Aldrich) were mixed at the ratio of 3:7 (volume ratio), and cultured for 24 hours under the conditions of 37°C and 5% CO₂. Next, Na tocopherol phosphate alone (final concentration of 10 µM in the culture medium), the inositol derivative alone (final concentration of 0.001% by mass in the culture medium), of a combination of Na tocopherol phosphate (final concentration of 10 µM in the culture medium) and the inositol derivative (final concentration of 0.001% by mass in the culture medium) was added to the culture medium. The Na tocopherol phosphate and the inositol derivative were dissolved in an aqueous solution of 0.05% (V/V) ethanol and added to the culture medium so that the final concentrations were as described above. Furthermore, one in which an aqueous solution of 0.05% (V/V) ethanol was added to the culture medium was also prepared. Thereafter, culturing was performed for 24 hours under the conditions of 37°C and 5% CO₂. Thereafter, 0.1 mL of a DMSO solution of atmospheric dust (NIST 1648a) was added per 100 mL of the culture medium so that the final concentration of the atmospheric dust in the culture medium was 500 µg/mL, and culturing was further performed for 48 hours. Thereafter, the culture medium was replaced with a medium containing 10% (V/V) of WST-8 of Nacalai Tesque Inc., and after further culturing for 3 hours, the absorbance at the wavelength of 450 nm was measured with a microplate reader i-Control (manufactured by Tecan Group Ltd.). One to which atmospheric dust was not added was used as a control.

Table 7 shows the results. Table 7 shows the cell proliferation amount in the atmospheric dust-added group as a relative amount when the absorbance in the control to which atmospheric dust was not added was 1. In any of the group in which the inositol derivative was added alone, the group in which the Na tocopherol phosphate was added alone, and the inositol derivative + Na tocopherol phosphate-added group, the cell proliferation amount was reduced as compared to the 0.05% ethanol-added group. In the inositol derivative + Na tocopherol phosphate-added group, the cell proliferation amount was reduced than the group in which the inositol derivative was added alone and the group in which the Na tocopherol phosphate was added alone. From these results, it was confirmed that the inositol derivative synergistically suppresses the cell proliferation of cancer cells accelerating due to atmospheric dust by using the inositol derivative together with Na tocopherol phosphate.

**[Table 7]**

| Sample | | Relative cell proliferation amount |
|---|---|---|
| Atmospheric dust not added | 0.05% Ethanol | 1.00 |
| Atmospheric dust added | 0.05% Ethanol | 1.69 |
| | Na tocopherol phosphate | 1.33 |
| | Inositol derivative | 1.42 |
| | Na tocopherol phosphate + inositol derivative | 0.94 |

### [Prescription examples]

Prescription examples of the composition for suppressing proliferation of cancer cells are described below. As an inositol derivative in the following prescription examples, the inositol derivative manufactured in [Manufacturing example of inositol derivative] is the exemplary example. Furthermore, as the following Na tocopherol phosphate, TPNa (registered trademark) (manufactured by Showa Denko K.K.), which is sodium dl-α-tocopheryl phosphate, is the exemplary example.

### (Prescription Example 1)

Table 8 shows prescription examples of a spreading agent (spray).

**[Table 8]**

| Component | Prescription (mass%) | |
|---|---|---|
| | Prescription Example 1-1 | Prescription Example 1-2 |
| Inositol derivative | 0.5 | 0.5 |
| Na tocopherol phosphate | - | 0.2 |
| Carboxy vinyl polymer | 1 | 1 |
| Ethyl alcohol | 30 | 30 |
| Phenoxyethanol | 0.2 | 0.2 |
| Water | 68.3 | 68.1 |

### (Prescription Example 2)

Table 9 shows prescription examples of a spreading agent (aerosol).

**[Table 9]**

| Component | Prescription (mass%) | |
|---|---|---|
| | Prescription Example 2-1 | Prescription Example 2-2 |
| Inositol derivative | 1 | 0.5 |
| Na tocopherol phosphate | - | 0.2 |
| Ethyl alcohol | 35 | 35 |
| Water | 10 | 10 |
| Nitrogen gas (propellant) | 54 | 54.3 |

### (Prescription Example 3)

Table 10 shows prescription examples of a spreading agent (spray for skin).

**[Table 10]**

| Component | Prescription (mass%) | |
|---|---|---|
| | Prescription Example 3-1 | Prescription Example 3-2 |
| Inositol derivative | 1 | 0.5 |
| Na tocopherol phosphate | - | 0.2 |
| Carboxy vinyl polymer | 1 | 1 |
| Ethyl alcohol | 25 | 25 |
| Titanium oxide | 0.5 | 0.5 |
| Sodium hyaluronate | 0.5 | 0.5 |
| Phenoxyethanol | 0.2 | 0.2 |
| Water | 71.8 | 72.1 |

### (Prescription Example 4)

Table 11 shows prescription examples of a nasal drop.

**[Table 11]**

| Component | Prescription (mass%) | |
|---|---|---|
| | Prescription Example 4-1 | Prescription Example 4-2 |
| Inositol derivative | 0.5 | 0.5 |
| Na tocopherol phosphate | - | 0.2 |
| Carboxy vinyl polymer | 0.5 | 0.5 |
| Sodium chloride | 1 | 1 |
| Benzalkonium chloride | 0.2 | 0.2 |
| Ethanol | 0.1 | 0.1 |
| Edetic acid | 0.2 | 0.2 |
| Menthol | Minute amounts | Minute amounts |
| Sorbitan sesquioleate | 0.2 | 0.2 |
| Water | 96.8 | 96.6 |

### [Industrial Applicability]

According to the present invention, a cancer cell proliferation suppression agent which can suppress proliferation of cancer cells accelerating due to atmospheric pollutants, and a composition for suppressing proliferation of cancer cells containing the above-mentioned cancer cell proliferation suppression agent are provided.

## Claims

1. An inositol derivative in which glucose or an oligosaccharide containing glucose as a constitutional unit is bound to inositol for use in suppressing cancer cell proliferation in a patient.

2. The inositol derivative for use in suppressing cancer cell proliferation in a patient according to Claim 1, wherein the inositol is myo-inositol.

3. The inositol derivative for use in suppressing cancer cell proliferation in a patient according to Claim 1 or 2, wherein the inositol derivative suppresses expression of a CYP1A1 gene and a CYP1B1 gene.

4. The inositol derivative for use in suppressing cancer cell proliferation in a patient according to any one of Claims 1 to 3, wherein the inositol derivative suppresses expression of an ARNT gene.

5. The inositol derivative for use in suppressing cancer cell proliferation in a patient according to any one of Claims 1 to 4, wherein the inositol derivative suppresses production of active oxygen.

6. A composition comprising the inositol derivative described in any one of Claims 1 to 5 and a pharmaceutically acceptable carrier for use in suppressing cancer cell proliferation in a patient.

7. The composition for use in suppressing cancer cell proliferation in a patient according to Claim 6, wherein a total content of the inositol derivative is 0.1% to 2% by mass.

8. The composition for use in suppressing cancer cell proliferation in a patient according to Claim 6 or 7, further comprising a tocopherol phosphate ester or a salt thereof.

9. The composition for use in suppressing cancer cell proliferation in a patient according to Claim 8, wherein the tocopherol phosphate ester is an α-tocopherol phosphate ester.

10. The composition for use in suppressing cancer cell proliferation in a patient according to Claim 8 or 9, wherein the salt of the tocopherol phosphate ester is a sodium salt of the tocopherol phosphate ester.

11. The composition for use in suppressing cancer cell proliferation in a patient according to any one of Claims 8 to 9, wherein a total content of the tocopherol phosphate ester or the salt thereof is 0.1% to 2% by mass.

## Patentansprüche

1. Inositolderivat, in dem Glucose oder ein Glucose als Grundeinheit enthaltendes Oligosaccharid an Inositol gebunden ist, zur Verwendung bei der Unterdrückung der Krebszellproliferation in einem Patienten.

2. Inositolderivat zur Verwendung bei der Unterdrückung der Krebszellproliferation bei einem Patienten nach Anspruch 1, wobei das Inositol Myo-Inositol ist.

3. Inositolderivat zur Verwendung bei der Unterdrückung der Krebszellproliferation in einem Patienten nach Anspruch 1 oder 2, wobei das Inositolderivat die Expression eines CYP1A1-Gens und eines CYP1B1-Gens unterdrückt.

4. Inositolderivat zur Verwendung bei der Unterdrückung der Krebszellproliferation in einem Patienten nach einem der Ansprüche 1 bis 3, wobei das Inositolderivat die Expression eines ARNT-Gens unterdrückt.

5. Inositolderivat zur Verwendung bei der Unterdrückung der Krebszellproliferation bei einem Patienten nach einem der Ansprüche 1 bis 4, wobei das Inositolderivat die Produktion von aktivem Sauerstoff unterdrückt.

6. Zusammensetzung, die das in einem der Ansprüche 1 bis 5 beschriebene Inositolderivat und einen pharmazeutisch annehmbaren Träger zur Verwendung bei der Unterdrückung der Krebszellproliferation in einem Patienten umfasst.

7. Zusammensetzung zur Verwendung bei der Unterdrückung der Krebszellproliferation bei einem Patienten nach Anspruch 6, wobei der Gesamtgehalt des Inositolderivats 0,1 bis 2 Massenprozent beträgt.

8. Zusammensetzung zur Verwendung bei der Unterdrückung der Krebszellproliferation in einem Patienten nach Anspruch 6 oder 7, die ferner einen Tocopherolphosphatester oder ein Salz davon umfasst.

9. Zusammensetzung zur Verwendung bei der Unterdrückung der Krebszellproliferation bei einem Patienten nach Anspruch 8, wobei der Tocopherolphosphatester ein α-Tocopherolphosphatester ist.

10. Zusammensetzung zur Verwendung bei der Unterdrückung der Krebszellproliferation in einem Patienten nach Anspruch 8 oder 9, wobei das Salz des Tocopherolphosphatesters ein Natriumsalz des Tocopherolphosphatesters ist.

11. Zusammensetzung zur Verwendung bei der Unterdrückung der Krebszellproliferation bei einem Patienten nach einem der Ansprüche 8 bis 9, wobei der Gesamtgehalt des Tocopherolphosphatesters oder des Salzes davon 0,1 bis 2 Massenprozent beträgt.

## Revendications

1. Dérivé d'inositol dans lequel du glucose ou un oligosaccharide contenant du glucose en tant qu'unité constitutive est lié à de l'inositol pour une utilisation dans la suppression de la prolifération de cellules cancéreuses chez un patient.

2. Dérivé d'inositol pour une utilisation dans la suppression de la prolifération de cellules cancéreuses chez un patient selon la revendication 1, dans lequel l'inositol est du myo-inositol.

3. Dérivé d'inositol pour une utilisation dans la suppression de la prolifération de cellules cancéreuses chez un patient selon la revendication 1 ou 2, dans lequel le dérivé d'inositol supprime l'expression d'un gène CYP1A1 et d'un gène CYP1B1.

4. Dérivé d'inositol pour une utilisation dans la suppression de la prolifération de cellules cancéreuses chez un patient selon l'une quelconque des revendications 1 à 3, dans lequel le dérivé d'inositol supprime l'expression d'un gène ARNT.

5. Dérivé d'inositol pour une utilisation dans la suppression de la prolifération de cellules cancéreuses chez un patient selon l'une quelconque des revendications 1 à 4, dans lequel le dérivé d'inositol supprime la production d'oxygène actif.

6. Composition comprenant le dérivé d'inositol décrit dans l'une quelconque des revendications 1 à 5 et un vecteur pharmaceutiquement acceptable pour une utilisation dans la suppression de la prolifération de cellules cancéreuses chez un patient.

7. Composition pour une utilisation dans la suppression de la prolifération de cellules cancéreuses chez un patient selon la revendication 6, dans laquelle une teneur totale du dérivé d'inositol est de 0,1 % à 2 % en masse.

8. Composition pour une utilisation dans la suppression de la prolifération de cellules cancéreuses chez un patient selon la revendication 6 ou 7, comprenant en outre un ester de phosphate de tocophérol ou un sel de celui-ci.

9. Composition pour une utilisation dans la suppression de la prolifération de cellules cancéreuses chez un patient selon la revendication 8, dans laquelle l'ester de phosphate de tocophérol est un ester de phosphate d'α-tocophérol.

10. Composition pour une utilisation dans la suppression de la prolifération de cellules cancéreuses chez un patient selon la revendication 8 ou 9, dans laquelle le sel de l'ester de phosphate de tocophérol est un sel de sodium de l'ester de phosphate de tocophérol.

11. Composition pour une utilisation dans la suppression de la prolifération de cellules cancéreuses chez un patient selon l'une quelconque des revendications 8 ou 9, dans laquelle une teneur totale de l'ester de phosphate de tocophérol ou du sel de celui-ci est de 0,1 % à 2 % en masse.
